# EUROPEAN PATENT APPLICATION

(11) **EP 3 705 086 A1**
(43) Date of publication of application: **09.09.2020**
(21) Application number: 19193787.9
(22) Date of filing: 27.08.2019
(51) Int. Cl.: A61F 2/00, A61L 27/36, A61L 27/58, A61L 27/56, A61F 2/08

(54) **IMPLANTABLE TISSUE STABILIZING STRUCTURE FOR IN SITU MUSCLE REGENERATION**

(30) Priority: 28.08.2018 US 201816114322; 24.08.2019 US 201916550228
(71) Applicant: Incorpracyl Technologies Ltd., 9644009 Jerusalem (IL)
(72) Inventor: Gorman, Joel, 9644009 Jerusalem (IL)
(74) Representative: Kretschmann, Dennis

(57) **Abstract**

An implantable tissue stabilizing structure (1) for regenerating damaged muscle in situ by enabling mass migration of muscle precursor cells into the damaged muscle. The structure is formed by a plurality of singular monofilament thread sections (11), which are separated by a plurality of void spaces (12) that define linear distances (13) between the threads. The maximal diameter of the threads is proportional to the linear distance, such that for linear distance less than 1 millimeter the maximal threads diameter is 40 microns, for linear distance from 1 to 2 millimeters the maximal diameter is 120 microns, for linear distance from 2 to 5 millimeters the maximal diameter is 400 microns, for linear distance from 10 to 20 millimeters the maximal diameter is 2.5 millimeters, and for linear distance of 40 millimeters and greater the thread sections maximal diameter is 10 millimeters.

## Description

### FIELD OF THE DISCLOSED TECHNIQUE

The disclosed technique relates to implantable tissue stabilizing structure for regenerating damaged muscle in situ by enabling mass migration of muscle precursor cells into the damaged muscle and methods.

### BACKGROUND OF THE DISCLOSED TECHNIQUE

In general, there is a need for healing damaged muscles by enabling muscle regeneration in *situ.* The present patent application discloses an implantable tissue stabilizing structure for regenerating damaged muscle in *situ* by enabling mass migration of muscle precursor cells into the damaged muscle and methods.

### DESCRIPTION OF THE DRAWINGS

The drawings attached to the application are not intended to limit the scope of the invention and the possible ways of its application. The drawings are intended only to illustrate the invention and constitute only one of many possible ways of its application.
FIG. 1 illustrates the implantable tissue stabilizing structure (1) that includes plurality of monofilament thread sections (11) that are separated by plurality of void spaced (12) that define a linear distance (13).
FIG. 2 illustrates the diameter (14) of the monofilament thread sections (12) and a precursor cell (2).

### THE INVENTION

The main object of the present invention is to provide an implantable tissue stabilizing structure for regenerating damaged muscle *in situ* by enabling mass migration of muscle precursor cells into the damaged muscle and methods.

The disclosed technique refers to an implantable tissue stabilizing structure that enables treating ventral hernias by functional restoration of the abdominal wall muscles in situ. This is achieved by a known biological principle combined with the innovative aspect of the invention subject matter of the present patent application. The known principle is that singular smooth monofilament thread sections in and of themselves are inert, neither stimulating substantial biologic response nor inhibiting innate spontaneous muscle regeneration. This principle is based on the universal experience with common monofilament surgical sutures.

The innovative aspect of the present application utilized in the disclosed technique is that when monofilament thread sections are arranged as a two-dimensional grid- or net-like structure (not necessarily of squares), the cross-sectional diameter of the singular monofilament thread sections will not present an insurmountable obstacle to migrating muscle precursor cells, as long as the cross-sectional diameter has a certain proportional relationship to the linear distance that the precursor cells must cross between thread sections, as defined below. The implantable tissue stabilizing structure initially provides mechanical stabilization of the herniating abdominal wall muscles, which is a basic condition for enabling spontaneous muscle regeneration. The open gap of the hernia and the intermittent protrusion through it of abdominal contents under high pressure physically inhibit muscle regeneration. The stabilization done by the structure comes to mechanically overcome these physical impediments to spontaneous muscle regeneration.

The implantable tissue stabilizing structure of the present invention is designed for regenerating damaged muscle in situ. This is achieved due to the fact that the structure provides the initial stabilization, as explained above, and by enabling the spontaneous muscle regeneration. This spontaneous muscle regeneration is achieved due to certain proportions of the structure as will be explained hereinafter.

The tissue stabilizing structure is formed by a plurality of singular monofilament thread sections that are separated by a plurality of void spaces. The void spaces define a linear distance between those thread sections. The structure enables spontaneous muscle regeneration by enabling mass migration of muscle precursor cells into the damaged muscle when the certain rules of proportionality between the diameter of the threads and the linear distance is:
(a) When the linear distance is less that 1 millimeter the cross-section diameter of the thread sections should be 40 microns or less.
(b) When the linear distance is from 1 to 2 millimeters the maximal cross-section diameter of the thread sections should be 120 microns.
(c) When the linear distance is from 2 to 5 millimeters the maximal cross-section diameter of the thread sections should be 400 microns.
(d) When the linear distance is from 5 to 10 millimeters the maximal cross-section diameter of the thread sections should be 1.4 millimeters.
(e) When the linear distance is from 10 to 20 millimeters the maximal cross-section diameter of the thread sections should be 2.5 millimeters.
(f) When the linear distance is from 20 to 40 millimeters the maximal cross-section diameter of the thread sections should be 5.0 millimeters, and
(g) When the linear distance is 40 millimeters or greater the thread sections maximal cross-sectional diameter is 10 millimeters.

The basic prior art requires mechanical stabilization of the hernia tissues, using a mesh, but differs by being based on the paradigm that a mesh will become fully integrated and mechanically strengthen the tissues by engendering collagen deposition and scar tissue formation. However, in addition to causing frequent complications, collagen deposition and scar tissue are known to be potent inhibitors of precursor cell infiltration and muscle regeneration. In contradistinction to this, the disclosed technique does not substantially inhibit innate spontaneous muscle regeneration. The long-term result of this unique capacity for initially stabilizing defective muscular tissues while preserving spontaneous self-regeneration is that the disclosed technique is buried within a fully regenerated mass of functional abdominal muscle. Because the buried structure remains as non-reactive and as flexible as when implanted, it has substantially no long-term direct physiological consequences, neither positive nor negative. However, it is responsible indirectly for a most physiological healing of ventral hernias, by enabling functional muscle and aponeurosis restoration through self-regeneration.

The underlying principles of the disclosed technique will be described in detail, as follows. The first principle is the non-interference of singular smooth monofilament thread sections in biologic processes. Such threads are inert due to the absence of micro-crevices. Micro-crevices occur only when threads, albeit technically "monofilament", are not singular and substantially isolated one from another, but instead are integrally intertwined as with knitting or knotting. At the nano-level this causes physicochemical destruction of the thread surfaces, and initiates protein adherence that triggers inflammation. On the micro-level, the complex arrangement of micro-crevices between threads produced by warf or weft knitting, as a common example, physically hinders cell movement and prevents scavenger cells from clearing debris and bacteria. This augments the inflammatory response causing a degree of hyper-fibrosis and scar tissue formation. Collagen deposition occurs early following implantation of knitted threads and is the critical event which immediately acts as a potent inhibitor of muscle precursor cell migration. Histological examination of the tissue reaction to state of the art meshes, both experimentally and clinically, never reveals any regenerated muscle fibers. The disclosed technique develops the state of the art by enabling spontaneous migration of substantial amount of myoblasts into damaged hernia tissues, leading to regeneration in situ of volumetric amounts of functional abdominal wall muscle and aponeuroses.

Before describing in detail the second, salient biological principle embodied in the disclosed technique, the utilized necessary mechanical technique will be detailed. Mechanical stabilization of the defective abdominal wall tissues, which secures neutralization of the open hernia gap and of the high-pressure protrusions through it, is achieved immediately upon implantation of the disclosed technique structure, due to its integral two-dimensional net-like structure. This gives the disclosed technique a mechanical role for bridging open hernia defects, and for reinforcing surgically closed ones, the latter being generally recognized as the recommended technique. From a mechanical perspective, the disclosed technique is suitable particularly for stabilizing closed tissues since the possibility then exists for incorporating larger void spaces which provides some advantage for enabling muscle regeneration. Nevertheless, the disclosed technique is not limited to relatively large void spaces, and necessary mechanical stabilization enabling muscle regeneration is obtained even with very small void spaces, as will be described below. Such delicate structures may indeed be suitable for enabling more delicate tissue regeneration. In any case, the innate mechanical strength of the disclosed technique is readily modulated, and depends on its constituent material. As examples, materials can be used which are quite rapidly absorbed, such as certain fibrin, collagen, hydrogel and alginate formulations. Alternatively, slowly absorbable synthetics can be used, such as P4HB (poly-4-hydroxybutarate). Materials of biological sources can be integrated as well, such as silk fibers or even substantially smooth products derived from mammalian origin such as muscle extracellular matrix. Non-absorbable synthetics, such as polypropylene and polyethylene, provide excellent strength and reliability, as well as low manufacturing cost. In all cases, the structural property which must be met is adequate strength until rapidly regenerating muscle and aponeurosis is well under way to restoring original abdominal wall strength, usually within 3 to 6 weeks of implantation.

Once the tissues to be repaired are stabilized mechanically by a structure which does not substantially interfere in biological cellular processes, it is possible for spontaneous muscle regeneration to proceed according to the unique second biological principle embodied in the disclosed technique. This most salient feature of the disclosed technique allows spontaneous regeneration by provision of a proportional relationship between the linear distance migrating myoblasts must cross between thread sections, and the cross-section diameter of the thread sections themselves. With the proper proportionality, the thread sections do not act as an insurmountable barrier, but rather enable continued spontaneous myloblast streaming (migration) and differentiation into elongated myotubes. Expressed in another way, the proper proportion between the void space distances and the potentially inhibiting structural elements prevents the latter from sending mechanobiological signals that arrest succeeding myoblast migration, and may even signal self-destruction of such cells. The goal of myoblast streaming in this context has been exceptionally well illustrated in the medical journal Stem Cell Research 30 (2018) :122-129, in which a photomicrograph is presented graphically showing myoblasts streaming or mass-migrating in huge numbers, generally aligned in a single direction from minimally prepared skeletal muscle onto matrigel-coated dishes. Myoblast streaming is likely to be enabled similarly in situ from abdominal muscle surrounding hernia defects following surgical implantation of the stabilizing disclosed technique.

The actual dimensions of the void spaces themselves are not restricted by the principle of proportionality. To illustrate, the linear distance travelled by myoblasts streaming in unison across a rectangular void space may correspond to the short width, long length, or some angled linear distance in between. The proportion necessary to enable streaming is the relationship of the linear distance traversed by the migrating cells to the cross-section diameter of the encountered structural threads defining the linear distance. Thus, if myoblasts migrate in the direction across the width of the rectangular space, the maximal cross-section diameter of the thread sections defining this relatively short distance must be relatively thin if myoblast migration is to proceed without negative mechanobiological feedback. Conversely, if a longer linear distance is crossed in this rectangle, the threads defining this distance may be relatively thicker, and still enable unhindered streaming. However, the specific size of the void space is irrelevant. The independent variable in the defined proportionality principle is the linear distance, and not the thread diameter, since this conforms more naturally to structural design considerations, as well as to practical anisotropic considerations.

Finally, the unique proportionality principle will be explained in detail on the cellular level. Regenerating myoblasts are approximately 25 microns in size. When only one or a few such cells migrate a relatively short distance, for example less than a millimeter, before encountering a potential obstacle such as a thread section of the disclosed technique that is 250 microns in cross-section diameter, it is likely that the relatively wall-like thread section will arrest the few leading cells to arrive at it. Moreover, these relatively few leading cells will send a negative retrograde signal to succeeding cells in effect instructing them to stop, turn back or self-destruct, thus aborting the migration locally and consequently the local regenerative process. If such inhibitory feedback is reproduced in many such small void spaces over a large area, then certainly the overall regenerative process will be aborted. However, if the thread diameter is also 25 microns, the migration of the first few cells to arrive will proceed to migrate substantially unhindered, and, despite the small distance for maneuvering, the obstacle will be surmounted, perhaps associated with positive retrograde stimulation of succeeding migrating cells. Conversely to this example, if a large number of cells streaming in unison traverse a relatively longer linear distance of perhaps 15 millimeters before encountering a smooth monofilament thread section of 1 millimeter in cross-section diameter, the leading front's retrograde signal may be cautionary only, which may be interpreted by succeeding migrating cells to mean to prepare to surmount. In this way, given this proportionality, the succeeding cells prepare their cytoskeletons and locomotive machinery to elongate and flexibly deform to climb over the preceding wave of cells, and thus in essence begin to contribute to a rampart-like formation, which following succeeding waves of cells can then surmount and augment until even the relatively large original obstacle is fully overcome, with myoblasts migration and muscle regeneration proceeding onwards. Ultimately, myoblasts become committed to differentiating into myotubes, which then coalesce into myofibers which may be several centimeters in length, and which require neurovascularization for final functional muscle restoration. All of this is enabled by the implantation initially of the disclosed technique embodying the proportionality principle. We would like to explain that enabled regeneration of abdominal wall tissue often incudes as in integral part of it differentiation and regeneration of aponeurotic fascia.

Careful and extensive assessment of the experimental literature, along with biologic and clinical sense gleaned from extensive experience in reconstructive abdominal wall surgery using hand-woven darning techniques with singular monofilament surgical sutures, as well as anecdotal surgical and radiologic evidence of muscle regeneration in certain cases, has led the inventor to the certain proportions for use in the disclosed technique. These proportions are designed to provide sufficient abdominal wall stabilization while enabling unhindered spontaneous musculoaponeurotic regeneration. When the linear distance in the disclosed technique which migrating cells traverse between one defining thread and another is less than 1 millimeter, 1 to 2 millimeters, 2 to 5 millimeters, 5 to 10 millimeters, 10 to 20 millimeters, 20 to 40 millimeters, and 40 millimeters or greater, then the corresponding maximal cross-section diameter of the defining thread sections is 40 microns, 120 microns, 400 microns, 1.4 millimeters, 2.5 millimeters, 5.0 millimeters, and 10 millimeters, respectively. The proportionality constant is approximately 1. Thus for every increase in linear distance between the thread sections, the maximal allowable thread section diameter is increased approximately to the same proportional degree, it being understood that the specified thread section diameter is the maximum that will not prevent spontaneous myoblast migration and thus muscle regeneration. The maximal cross-section diameter of threads separated by the smaller distances is proportionally somewhat greater than for the longer distances. This is explained by the proportionally greater contribution of individual cell deformability when overcoming obstacles by cells migrating over relatively shorter distances. Conversely, myoblasts moving over longer distances rely on the relatively less efficient method for overcoming obstacles of succeeding waves of cells first surmounting "front line" cells.

An important clinical corollary of the proportionality principle is its practical suitability for clinical surgery. As evidenced by the photomicrograph referred to above of swarming myoblasts, muscle precursor cells are not a cell type rarely found in native skeletal muscle, but rather represents approximately 5% of all cell nuclei in normal muscle. Moreover, due to their presence in sub-laminal and sub-fascial niches, they are readily exposed and made available for a rapid massive response to tissue damage, including rapid proliferation and multiplication in number. Such tissue damage is inevitably brought about during surgical correction of hernias. Therefore, following implantation of the disclosed technique, muscle regeneration is well underway in a matter of days following surgery, and reaches functional completion within a matter of a few months at most. There are surgical techniques that are particularly amenable for using the disclosed technique to take advantage of this phenomenon. One such is the increasingly popular open anterior, or laparoscopic posterior, rectus sheath turnover flap technique, for which the disclosed technique is highly appropriate. This may help in finally allowing the "standardization of surgical technique in order to develop a reliable approach to hernia repair that can be offered to an increasing number of patients." (A. Carbonell; World Journal of Gastointestinal Surgery (2015) 7:293)

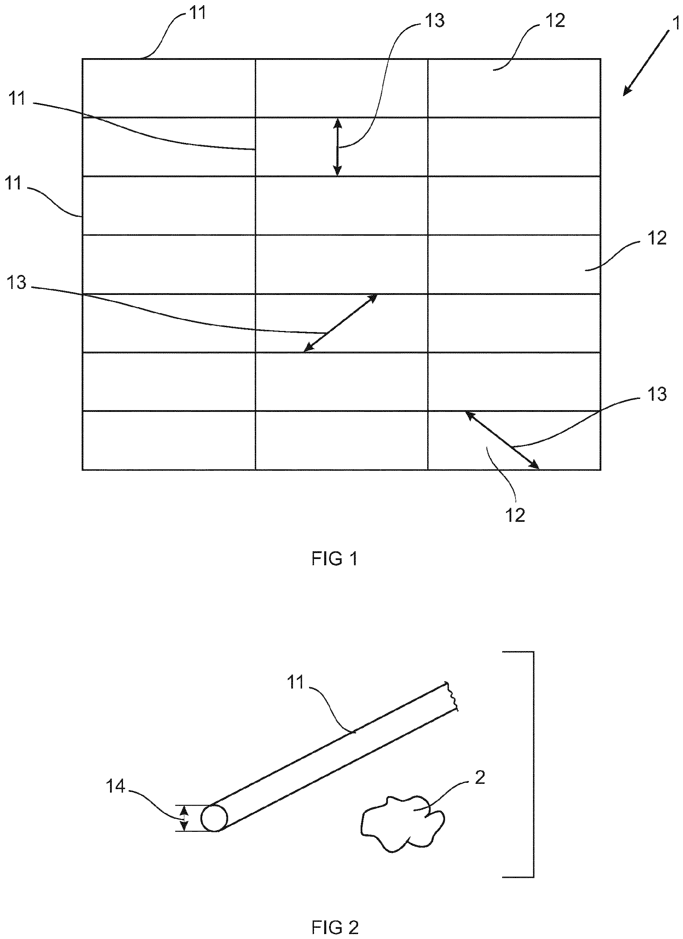

Figure 1 illustrates the implantable tissue stabilizing structure (1) that includes plurality of monofilament thread sections (11) that are separated by plurality of void spaces (12) that define linear distances (13). Figure 2 illustrates the diameter (14) of the monofilament thread sections (12) and a precursor cell (2).

The present invention refers also to methods that use the implantable tissue stabilizing structure (1) for regenerating damaged muscle *in situ.*

The first method is for regenerating damaged muscle in situ by enabling mass migration of muscle precursor cells (2) into the damaged muscle. The method includes the following:
(a) providing the implantable tissue stabilizing structure (1).
(b) fixating by open surgical techniques the implantable tissue stabilizing structure to defective musculoaponeurotic tissue.

By this method the fixation immediately provides mechanical stabilization of the tissue and enables migration of muscle precursor cells into the damaged muscle, leading to regeneration of volumetric amounts of functionalized musculoaponeurotic tissue.

The second method is for *in-situ* tissue engineering for surgical reconstruction of volumetric muscle loss using autologous tissues. The method includes the following:
(a) providing the implantable tissue stabilizing structure (1).
(b) fixating the implantable tissue stabilizing structure to a donor site comprising normal muscle tissue which has been prepared by intentional injury thereby enabling muscle tissues to regenerate spontaneously in and around the implantable tissue stabilizing structure.
(c) excising in toto from the donor site the implantable tissue stabilizing structure together with newly formed muscle tissue within and surrounding the implantable tissue stabilizing structure, and
(d) transplanting en masse the implantable tissue stabilizing structure together with the newly formed muscle tissue within and surrounding the implantable tissue stabilizing structure, to autologous recipient site of volumetric muscle loss. By this method the transplanted regenerated muscle tissue re-vascularizes and continues to regenerate and to produce volumetric amounts of regenerated autologous muscle tissue at the recipient site.

The present invention also discloses an implantable tissue stabilizing structure for regenerating damaged native tissue by enabling mass migration of native tissue precursor cells into the damaged native tissue; wherein said tissue stabilizing structure is a single layer structure formed by a plurality of monofilament or multifilament thread sections; wherein said thread sections are separated by a plurality of void spaces; wherein said void spaces define a linear distance between said thread sections; wherein the maximal cross-section diameter of said thread sections is proportional to said linear distance, such that for said linear distance less than 5 microns said maximal thread diameter is 200 nanometers, for said linear distance from 5 to 40 microns said maximal thread diameter is 1 micron, for said linear distance from 40 to 200 microns said maximal thread diameter is 10 microns, for said linear distance from 200 microns to 1 millimeter said maximal thread diameter is 40 microns, for said linear distance from 1 to 2 millimeters said maximal thread diameter is 120 microns, for said linear distance from 2 to 5 millimeters said maximal thread diameter is 400 microns, for said linear distance from 5 to 10 millimeters said maximal thread diameter is 1.4 millimeters, for said linear distance from 10 to 20 millimeters said maximal thread diameter is 2.5 millimeters, for said linear distance from 20 to 40 millimeters said maximal thread diameter is 5.0 millimeters, and for said linear distance of 40 millimeters and greater said thread sections maximal cross-sectional diameter is 10 millimeters.

The present invention also discloses a method for regenerating damaged native tissue in situ by enabling mass migration of native tissue precursor cells into the damaged native tissue, comprising:
(a) providing an implantable tissue stabilizing structure that is a single layer structure formed by a plurality of monofilament or multifilament thread sections, wherein said thread sections are separated by a plurality of void spaces, wherein said void spaces define a linear distance between said thread sections, wherein the maximal cross-section diameter of said thread sections is proportional to said linear distance, such that for said linear distance less than 5 microns said maximal thread diameter is 200 nanometers, for said linear distance from 5 to 40 microns said maximal thread diameter is 1 micron, for said linear distance from 40 to 200 microns said maximal thread diameter is 10 microns, for said linear distance from 200 microns to 1 millimeter said maximal thread diameter is 40 microns, for said linear distance from 1 to 2 millimeters said maximal thread diameter is 120 microns, for said linear distance from 2 to 5 millimeters said maximal thread diameter is 400 microns, for said linear distance from 5 to 10 millimeters said maximal thread diameter is 1.4 millimeters, for said linear distance from 10 to 20 millimeters said maximal thread diameter is 2.5 millimeters, for said linear distance from 20 to 40 millimeters said maximal thread diameter is 5.0 millimeters, and for said linear distance of 40 millimeters and greater said thread sections maximal cross-sectional diameter is 10 millimeters;
(b) fixating by open surgical and laparoscopic techniques said single-layer tissue stabilizing structure to defective native tissue; and
whereby said fixation immediately provides mechanical stabilization of said native tissue, and enables migration of precursor native tissue cells into said damaged native tissue, leading to regeneration of volumetric amounts of funtionalized native tissue.

The present invention also discloses a method of in situ tissue engineering for surgical reconstruction of volumetric muscle loss using autologous tissues, comprising:
(a) providing an implantable tissue stabilizing structure that is a single layer structure formed by a plurality of monofilament or multifilament thread sections, wherein said thread sections are separated by a plurality of void spaces, wherein said void spaces define a linear distance between said thread sections, wherein the maximal cross-section diameter of said thread sections is proportional to said linear distance, such that for said linear distance less than 5 microns said maximal thread diameter is 200 nanometers, for said linear distance from 5 to 40 microns said maximal thread diameter is 1 micron, for said linear distance from 40 to 200 microns said maximal thread diameter is 10 microns, for said linear distance from 200 microns to 1 millimeter said maximal thread diameter is 40 microns, for said linear distance from 1 to 2 millimeters said maximal thread diameter is 120 microns, for said linear distance from 2 to 5 millimeters said maximal thread diameter is 400 microns, for said linear distance from 5 to 10 millimeters said maximal thread diameter is 1.4 millimeters, for said linear distance from 10 to 20 millimeters said maximal thread diameter is 2.5 millimeters, for said linear distance from 20 to 40 millimeters said maximal thread diameter is 5.0 millimeters, and for said linear distance of 40 millimeters and greater said thread sections maximal cross-sectional diameter is 10 millimeters;
(b) fixating said implantable tissue stabilizing structure to a donor site comprising normal muscle tissue which has been prepared by intentional injury thereby enabling muscle tissue to regenerate spontaneously in and around said implantable tissue stabilizing structure;
(c) excising in toto from said donor site said implantable tissue stabilizing structure together with newly formed muscle tissue within and surrounding said implantable tissue stabilizing structure;
(d) transplanting en masse said implantable tissue stabilizing structure together with said newly formed muscle tissue within and surrounding said implantable tissue stabilizing structure, to autologous recipient site of volumetric muscle loss; and
whereby the transplanted regenerated muscle tissue re-vascularizes and continues to regenerate and to produce volumetric amounts of regenerated muscle tissue at the recipient site.

### A native tissue

It should be noted that the implantable tissue supporting structure described above and herein is designed to enable spontaneous regeneration of native tissues in general. Muscle tissue, referred to above, includes skeletal, smooth, and cardiac muscle, and thus is by far the most prevalent human tissue. Therefore, injured muscle tissue in general, and hernia defects of abdominal wall muscle tissue in particular, are the most common target tissue for state of the art areal implants, and thus suitably represent the various types of injured tissue that will benefit from the disclosed technique. Accordingly, the disclosed technique is designed, for example, to enable regeneration of epithelial tissue and smooth muscle for esophageal and tracheal reconstruction, epidermal skin tissue, and osseous tissue for orbital and craniomaxillofacial reconstruction; to provide stabilization of highly focused areas of uterine lining and a vehicle for controlled oocyte implantation; for healing of myocardial infarctions by a unique cardiac patch, as well as for skeletal muscle regeneration for healing ventral hernias, as referred to above.

### A single-layer

It should also be noted that the implantable stabilizing structure described above and herein is essentially a two-dimensional structure composed of a distinct single layer of thread sections. This is to be distinguished from seemingly two-dimensional structures of the prior art which are actually composed of multiple layers of thread sections. Common examples include electrospun nano-fibrous structures, and biological scaffolds composed of decellularized extracellular matrix. These appear to be two-dimensional single-layer structures on gross inspection, but on microscopic examination are revealed to be deep three-dimensional multi-layered jumbles of intertwined thread-like sections. This in fact constitutes their purported advantage, as they seem to mimic the architectural structure of extracellular matrix found in living tissue - purportedly allowing regenerating precursor cells to "feel at home" to infiltrate, differentiate and multiply into native tissue. Unfortunately this never occurs, the sole reason being the absence of that specific proportionality between the void space and thread dimensions necessary for enabling neo-formation of extracellular matrix as an integral part of overall native tissue regeneration. In this regard, it is adequate for enabling complete functional native tissue regeneration that the disclosed technique embodies only a single layer of sufficiently strong thread sections that uniquely reflect the proportionality structure as described above and herein.

### Multifilaments

As described above, the disclosed technique employs the known biological principle that singular monofilament thread sections are substantially biologically inert. However, it should also be noted that either monofilament or multifilament thread sections, tightly adjoined or braided, or closely intertwined, also approach adequate biological inertness. Therefore, as long as the unique second principle of proper proportionality is met, spontaneous native tissue regeneration is enabled by an implantable single-layer tissue support structure composed of multifilament threads.

The cross-section diameters of such multifilamentous thread sections are subject to the same proportionalities to the linear distances separating them, as are singular monofilament thread sections. However, they are measured somewhat differently, as the height between, inclusively, the uppermost and lowermost filaments perpendicular to the linear distance. In this regard, it should be noted that the cross-section diameter of singular monofilament thread sections is likewise measured perpendicular to the linear distance.

### Microscopic embodiments

The current paradigm in the prior art states that the smaller the void spaces of an implantable mesh, matrix or scaffold, the less likely it is that the cellular responses to it will be anything other than foreign-body inflammation and scar tissue formation, and least likely of all native tissue regeneration. However, according to the proportionality structure of the disclosed technique, spontaneous native tissue regeneration is as likely to occur through and around microscopic void spaces as it is through macro-pores. That is, the *sine qua non* for enabling spontaneous native tissue regeneration is simply to embody the proportionality structure described above. However, the proportionality structure of the disclosed technique enables spontaneous native tissue regeneration equally well in relation to microscopic void spaces and macropores. Since this is counter-intuitive and against the current paradigm, it deserves further explanation.

It has already been described that the disclosed technique is not limited to relatively large void spaces, and in fact the specific size of the void space is irrelevant, and the actual dimensions of the void spaces themselves are not restricted by the structure of proportionality. Moreover, as already stated, necessary mechanical stabilization enabling muscle regeneration is obtained even with very small void spaces, and such delicate structures may indeed be suitable for enabling more delicate tissue regeneration.

Therefore, it should also be noted that void spaces well under 1 millimeter in linear distance enable spontaneous native tissue regeneration, as long as the proportionality structure is respected. In light of their counter-intuitive nature, those embodiments of the disclosed technique with linear distances less than 1 millimeter are detailed here as follows: embodiments whose thread sections are separated by linear distances of up to 5 microns, 5 to 40 microns, 40 to 200 microns, and 200 microns to 1 millimeter, are all capable of enabling native tissue regeneration when the maximal cross-section thread diameter is 200 nanometers, 1 micron, 10 microns, and 40 microns, respectively.

All these categories of embodiments with microscopic dimensions manifest the counter-intuitive property of enabling spontaneous regeneration despite having microscopic void spaces. The biological mechanisms underlying this property can be explained as follows. In addition to the previously described mechanisms of rampart-building by succeeding waves of streaming precursor cells, and of individual precursor cell deformability, embodiments with microscopic dimensions reflect another underlying mechanism that enables native tissue regeneration. The relative importance of this mechanism increases with decreasing dimensions. The mechanism regards transduction of mechanobiological signals from exceedingly thin thread section obstacles, for example 150 nanometers in diameter, which despite their small size provide necessary mechanical stabilization to the injured native tissue. The mechanobiological signals from them are transduced to the individual migrating cell with a frequency dependent on the sub-cellular distances(e.g. 3 microns) separating the thread-like obstacles and the velocity of migration, much like road-bumps transmitted to a car's occupants. The transmission of signals from such highly localized sources to an individual cell is via correspondingly localized parts of the cell's wall, with the message ultimately reaching locomotive proteins in the cytoplasm. When such transmission is weak enough that cell motility is not aborted, but strong enough to trigger movement over the obstacles, then spontaneous regeneration proceeds and the local tissue damage is naturally and fully repaired. Such balanced and effective signal transduction for single-layer implants of the smallest dimensions simply depends on their having the proper proportionality between cross-section diameters and linear distances, as enunciated above.

It is recognized that embodiments with micro- and nano-scopic dimensions require special nanotechnology techniques for manufacturing, such as 3D nano-printing, as well as micro- and nano-surgical techniques for implantation. For the purpose of orientation, it can be noted that monofilament sutures are currently produced down to 20 micron diameter. In the very lowest range, the implants are not visible to the naked eye. However, with the proper proportionality between the microscopic distances separating the threads and the threads' microscopic and nano-scopic diameters, implantation into highly focal areas of damaged tissue gives adequate mechanical stability and enables healing by highly localized spontaneous native tissue regeneration.

### Spherical

It should also be noted that an implantable single-layer stabilizing structure may lack borders and still substantially manifest the basic two-dimensional proportionalities as delineated above for enabling regeneration. This is so when such a single layer curves back on itself, in essence forming a completely hollow structure defined solely by its single-layer outer surface. When the two-dimensional quality of this outer surface includes the quantitative proportionalities enunciated above, such hollow structures uniquely enable migrating native tissue precursor cells to stream through and around the outer surface layer, and fill the hollow internal space with regenerating native tissue. In accordance with this, it is seen that such hollow structures of any shape and size but especially of microscopic dimensions (e.g. 1 mm diameter) permit relatively volumetric regeneration of localized, even microscopic areas of damaged tissues, when they are applied by manual implantation, injection, flow or sprinkling (as from a salt shaker). Furthermore, this unique means of enabling native tissue regeneration can be expanded or facilitated by joining pro-regenerative molecular substances to the preformed hollow micro-pellets prior to their application.

### in situ

It also be noted that the technique disclosed above enables tissue regeneration not only *in situ,* but *ex vivo* and *in vitro* as well. For example, laboratory studies of tissue engineering are improved when mechanically supportive structures are utilized that are not inhibitory of *in vitro* tissue regeneration as in the state of the art, but rather uniquely enable it as with the disclose technique.

## Claims

1. An implantable tissue stabilizing structure for regenerating damaged native tissue by enabling mass migration of native tissue precursor cells into the damaged native tissue; wherein said tissue stabilizing structure is a single layer structure formed by a plurality of monofilament or multifilament thread sections; wherein said thread sections are separated by a plurality of void spaces; wherein said void spaces define a linear distance between said thread sections; wherein the maximal cross-section diameter of said thread sections is proportional to said linear distance, such that for said linear distance less than 5 microns said maximal thread diameter is 200 nanometers, for said linear distance from 5 to 40 microns said maximal thread diameter is 1 micron, for said linear distance from 40 to 200 microns said maximal thread diameter is 10 microns, for said linear distance from 200 microns to 1 millimeter said maximal thread diameter is 40 microns, for said linear distance from 1 to 2 millimeters said maximal thread diameter is 120 microns, for said linear distance from 2 to 5 millimeters said maximal thread diameter is 400 microns, for said linear distance from 5 to 10 millimeters said maximal thread diameter is 1.4 millimeters, for said linear distance from 10 to 20 millimeters said maximal thread diameter is 2.5 millimeters, for said linear distance from 20 to 40 millimeters said maximal thread diameter is 5.0 millimeters, and for said linear distance of 40 millimeters and greater said thread sections maximal cross-sectional diameter is 10 millimeters.

2. A method for regenerating damaged native tissue *in situ* by enabling mass migration of native tissue precursor cells into the damaged native tissue, comprising:
(a) providing an implantable tissue stabilizing structure that is a single layer structure formed by a plurality of monofilament or multifilament thread sections, wherein said thread sections are separated by a plurality of void spaces, wherein said void spaces define a linear distance between said thread sections, wherein the maximal cross-section diameter of said thread sections is proportional to said linear distance, such that for said linear distance less than 5 microns said maximal thread diameter is 200 nanometers, for said linear distance from 5 to 40 microns said maximal thread diameter is 1 micron, for said linear distance from 40 to 200 microns said maximal thread diameter is 10 microns, for said linear distance from 200 microns to 1 millimeter said maximal thread diameter is 40 microns, for said linear distance from 1 to 2 millimeters said maximal thread diameter is 120 microns, for said linear distance from 2 to 5 millimeters said maximal thread diameter is 400 microns, for said linear distance from 5 to 10 millimeters said maximal thread diameter is 1.4 millimeters, for said linear distance from 10 to 20 millimeters said maximal thread diameter is 2.5 millimeters, for said linear distance from 20 to 40 millimeters said maximal thread diameter is 5.0 millimeters, and for said linear distance of 40 millimeters and greater said thread sections maximal cross-sectional diameter is 10 millimeters;
(b) fixating by open surgical and laparoscopic techniques said single-layer tissue stabilizing structure to defective native tissue; and
whereby said fixation immediately provides mechanical stabilization of said native tissue, and enables migration of precursor native tissue cells into said damaged native tissue, leading to regeneration of volumetric amounts of functionalized native tissue.

3. A method of *in situ* tissue engineering for surgical reconstruction of volumetric muscle loss using autologous tissues, comprising:
(a) providing an implantable tissue stabilizing structure that is a single layer structure formed by a plurality of monofilament or multifilament thread sections, wherein said thread sections are separated by a plurality of void spaces, wherein said void spaces define a linear distance between said thread sections, wherein the maximal cross-section diameter of said thread sections is proportional to said linear distance, such that for said linear distance less than 5 microns said maximal thread diameter is 200 nanometers, for said linear distance from 5 to 40 microns said maximal thread diameter is 1 micron, for said linear distance from 40 to 200 microns said maximal thread diameter is 10 microns, for said linear distance from 200 microns to 1 millimeter said maximal thread diameter is 40 microns, for said linear distance from 1 to 2 millimeters said maximal thread diameter is 120 microns, for said linear distance from 2 to 5 millimeters said maximal thread diameter is 400 microns, for said linear distance from 5 to 10 millimeters said maximal thread diameter is 1.4 millimeters, for said linear distance from 10 to 20 millimeters said maximal thread diameter is 2.5 millimeters, for said linear distance from 20 to 40 millimeters said maximal thread diameter is 5.0 millimeters, and for said linear distance of 40 millimeters and greater said thread sections maximal cross-sectional diameter is 10 millimeters;
(b) fixating said implantable tissue stabilizing structure to a donor site comprising normal muscle tissue which has been prepared by intentional injury thereby enabling muscle tissue to regenerate spontaneously in and around said implantable tissue stabilizing structure;
(c) excising *in toto* from said donor site said implantable tissue stabilizing structure together with newly formed muscle tissue within and surrounding said implantable tissue stabilizing structure;
(d) transplanting *en masse* said implantable tissue stabilizing structure together with said newly formed muscle tissue within and surrounding said implantable tissue stabilizing structure, to autologous recipient site of volumetric muscle loss; and
whereby the transplanted regenerated muscle tissue re-vascularizes and continues to regenerate and to produce volumetric amounts of regenerated muscle tissue at the recipient site.

4. An implantable tissue stabilizing structure for regenerating damaged muscle *in situ* by enabling mass migration of muscle precursor cells into the damaged muscle; wherein said tissue stabilizing structure is formed by a plurality of singular monofilament thread sections; wherein said thread sections are separated by a plurality of void spaces; wherein said void spaces define a linear distance between said thread sections; wherein the maximal cross-section diameter of said thread sections is proportional to said linear distance, such that for said linear distance less than 1 millimeter said thread sections maximal cross-sectional diameter is 40 microns, for said linear distance from 1 to 2 millimeters said maximal thread diameter is 120 microns, for said linear distance from 2 to 5 millimeters said maximal thread diameter is 400 microns, for said linear distance from 5 to 10 millimeters said maximal thread diameter is 1.4 millimeters, for said linear distance from 10 to 20 millimeters said maximal thread diameter is 2.5 millimeters, for said linear distance from 20 to 40 millimeters said maximal thread diameter is 5.0 millimeters, and for said linear distance of 40 millimeters and greater said thread sections maximal cross-sectional diameter is 10 millimeters.

5. A method for regenerating damaged muscle *in situ* by enabling mass migration of muscle precursor cells into the damaged muscle, comprising:
(a) providing an implantable tissue stabilizing structure, wherein said tissue stabilizing structure is formed by a plurality of singular monofilament thread sections; wherein said thread sections are separated by a plurality of void spaces; wherein said void spaces define a linear distance between said thread sections; wherein the maximal cross-section diameter of said thread sections is proportional to said linear distance, such that for said linear distance less than 1 millimeter said thread sections maximal cross-sectional diameter is 40 microns, for said linear distance from 1 to 2 millimeters said maximal thread diameter is 120 microns, for said linear distance from 2 to 5 millimeters said maximal thread diameter is 400 microns, for said linear distance from 5 to 10 millimeters said maximal thread diameter is 1.4 millimeters, for said linear distance from 10 to 20 millimeter said maximal thread diameter is 2.5 millimeters, for said linear distance from 20 to 40 millimeters said maximal thread diameter is 5.0 millimeters, and for said linear distance of 40 millimeters and greater said thread sections maximal cross-sectional diameter is 10 millimeters;
(b) fixating by open surgical and laparoscopic techniques said implantable tissue stabilizing structure to defective musculoaponeurotic tissue; and
whereby said fixation immediately provides mechanical stabilization of said tissue, and enables migration of precursor muscle cells into said damaged muscle, leading to regeneration of volumetric amounts of functionalized musculoaponeurotic tissue.

6. A method of *in situ* tissue engineering for surgical reconstruction of volumetric muscle loss using autologous tissues, comprising:
(a) providing an implantable tissue stabilizing structure, wherein said tissue stabilizing structure is formed by a plurality of singular monofilament thread sections; wherein said thread sections are separated by a plurality of void spaces; wherein said void spaces define a linear distance between said thread sections; wherein the maximal cross-section diameter of said thread sections is proportional to said linear distance, such that for said linear distance less than 1 millimeter said thread sections maximal cross-sectional diameter is 40 microns, for said linear distance from 1 to 2 millimeters said maximal thread diameter is 120 microns, for said linear distance from 2 to 5 millimeters said maximal thread diameter is 400 microns, for said linear distance from 5 to 10 millimeters said maximal thread diameter is 1.4 millimeters, for said linear distance from 10 to 20 millimeter said maximal thread diameter is 2.5 millimeter, for said linear distance from 20 to 40 millimeters said maximal thread diameter is 5.0 millimeters, and for said linear distance of 40 millimeters and greater said thread sections maximal cross-sectional diameter is 10 millimeters;
(b) fixating said implantable tissue stabilizing structure to a donor site comprising normal muscle tissue which has been prepared by intentional injury thereby enabling muscle tissues to regenerate spontaneously in and around said implantable tissue stabilizing structure;
(c) excising *in toto* from said donor site said implantable tissue stabilizing structure together with newly formed muscle tissue within and surrounding said implantable tissue stabilizing structure;
(d) transplanting en masse said implantable tissue stabilizing structure together with said newly formed muscle tissue within and surrounding said implantable tissue stabilizing structure, to autologous recipient site of volumetric muscle loss; and
whereby the transplanted regenerated muscle tissue re-vascularizes and continues to regenerate and to produce volumetric amounts of regenerated muscle tissue at the recipient site.
